(19) Europäisches Patentamt
European Patent Office
Office européen des brevets

(11) Veröffentlichungsnummer: **0 045 430**
**A1**

(12) # EUROPÄISCHE PATENTANMELDUNG

(21) Anmeldenummer: **81105681.1**

(22) Anmeldetag: **20.07.81**

(51) Int. Cl.³: **C 07 C 25/13**
C 07 C 17/14

(30) Priorität: **01.08.80 DE 3029365**

(43) Veröffentlichungstag der Anmeldung:
**10.02.82 Patentblatt 82/6**

(84) Benannte Vertragsstaaten:
**BE CH DE FR GB IT LI**

(71) Anmelder: **BAYER AG**
**Zentralbereich Patente, Marken und Lizenzen**
**D-5090 Leverkusen 1, Bayerwerk(DE)**

(72) Erfinder: **Blank, Heinz Ulrich, Dr.**
**Am Geusfelde 35**
**D-5068 Odenthal(DE)**

(72) Erfinder: **Wolters, Erich, Dr.**
**Streffenweg 22**
**D-5162 Niederzier(DE)**

(72) Erfinder: **Rottloff, Günther**
**Semmelweisstrasse 70**
**D-5000 Koeln 80(DE)**

(72) Erfinder: **Diehl, Herbert, Dr.**
**Heymannstrasse 40**
**D-5090 Leverkusen 1(DE)**

(54) **Verfahren zur alpha-Bromierung von gegebenenfalls substituierten Fluortoluolen und Gemische von in alpha-Stellung verschieden hoch bromierten, gegebenenfalls substituierten Fluortoluolen.**

(57) Gegebenenfalls substituierte Fluortoluole werden in α-Stellung mit 0,5 bis 3,2 Mol Brom pro Mol Fluortoluol bei einer Temperatur von 130 bis 250°C und unter kontinuierlicher Entfernung des gleichzeitig gebildeten Bromwasserstoffs bromiert. Hierbei kann in Gegenwart eines Lösungsmittels oder unter photochemischer oder radikalischer Initiierung gearbeitet werden. Beim Einsatz verschiedener Brommengen innerhalb des angegebenen Bereiches können Gemische erhalten werden, die 90 bis 93 Gew.-% des entsprechenden Fluorbenzalbromids, 3 bis 4 Gew.-% des entsprechenden Fluorbenzylbromids und 4 bis 6 Gew.-% des entsprechenden Fluorbenzotribromids enthalten, oder Gemische, die 15 bis 95 Gew.-% des entsprechenden Fluorbenzotribromids und 5 bis 85 Gew.-% des entsprechenden Fluorbenzalbromids enthalten, oder Gemische, die 40 bis 85 Gew.-% des entsprechenden Fluorbenzalbromids und 15 bis 60 Gew.-% des entsprechenden Fluorbenzylbromids enthalten, oder Gemische, die 95 bis 100 Gew.-% des entsprechenden Fluorbenzalbromids und 0 bis 5 Gew.-% des entsprechenden Fluorbenzylbromids enthalten.

EP 0 045 430 A1

Croydon Printing Company Ltd.

BAYER AKTIENGESELLSCHAFT
Zentralbereich
Patente, Marken und Lizenzen

5090 Leverkusen, Bayerwerk
Ha/Zar

Verfahren zur α-Bromierung von gegebenenfalls substituierten Fluortoluolen und Gemische von in α-Stellung verschieden hoch bromierten, gegebenenfalls substituierten Fluortoluolen

Die Erfindung betrifft ein Verfahren zur α-Bromierung von gegebenenfalls substituierten Fluortoluolen und Gemische, die gegebenenfalls substituiertes Fluorbenzalbromid, gegebenenfalls substituiertes Fluorbenzylbromid und gegebenenfalls substituiertes Fluorbenzotribromid enthalten.

Es wurde ein Verfahren zur α-Bromierung von gegebenenfalls substituierten Fluortoluolen gefunden, das dadurch gekennzeichnet ist, daß man Fluortoluole der Formel

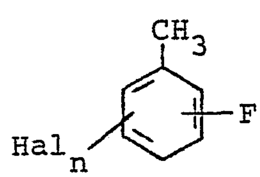

(I),

in der

Hal für Fluor, Chlor oder Brom steht und
n gleich 0 oder 1 ist,

mit 0,5 bis 3,2 Mol Brom pro Mol gegebenenfalls substituiertes Fluortoluol bei einer Temperatur von 130 bis 250°C, gegebenenfalls in Gegenwart eines Lösungsmittels und gegebenenfalls unter photochemischer oder radikalischer Initiierung umsetzt und den gleichzeitig gebildeten Bromwasserstoff kontinuierlich aus dem Reaktionsgemisch entfernt.

Erfindungsgemäß werden gegebenenfalls substituierte Fluortoluole der Formel (I) der α-Bromierung unterworfen. Als Halogen sei beispielsweise Fluor, Chlor oder Brom, bevorzugt Chlor oder Brom, besonders bevorzugt Brom, genannt.

Der Index n steht für die Zahl 0 oder 1, bevorzugt für die Zahl 1, so daß der Index dann entfallen kann.

Entsprechend werden bevorzugt Halogen-substituierte Fluortoluole der Formel

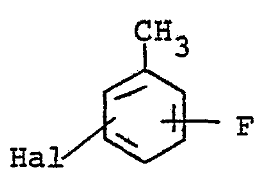

(II)

eingesetzt, in der Hal die oben gegebene Bedeutung hat.

Besonders bevorzugt werden erfindungsgemäß Brom-fluortoluole der Formel

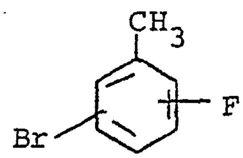

(III)

eingesetzt.

Ganz besonders bevorzugt wird erfindungsgemäß 3-Brom-4-fluorbenzol eingesetzt.

Erfindungsgemäß werden zur α-Bromierung 0,5 bis 3,2 Mol Brom pro Mol gegebenenfalls substituiertes Fluortoluol eingesetzt. Hierbei kann in Abhängigkeit von der eingesetzten Menge Brom innerhalb des beschriebenen Bereiches ein verschieden hoher Grad an α-Bromierung erreicht werden. So kann beispielsweise beim Einsatz von etwa 1,9 bis 2,2, vorzugsweise 2,0 bis 2,1, Mol Brom pro Mol gegebenenfalls substituiertes Fluortoluol ein Reaktionsgemisch erhalten werden, das etwa 90 bis 93 Gew.-% gegebenenfalls substituiertes Fluorbenzalbromid neben untergeordneten Mengen von gegebenenfalls substituiertem Fluorbenzylbromid, beispielsweise 3 bis 4 Gew.-% und von gegebenenfalls substituiertem Fluorbenzotribromid, beispielsweise 4 bis 6 Gew.-%, enthält. Alle Gewichtsprozente beziehen sich auf das Gesamtgewicht des bei der α-Bromierung erhaltenen Reaktionsgemisches.

Beim Einsatz von etwa 0,5 bis 1,9, bevorzugt 1,2 bis 1,8, besonders bevorzugt 1,5 bis 1,7, Mol Brom pro Mol gegebenenfalls substituiertes Fluortoluol können beispielsweise Bromierungsgemische erhalten werden,

in denen das zugehörige Benzotribromid unter 1 Gew.-%, bevorzugt unter 0,1 Gew.-%, enthalten ist. Beispielsweise kann beim Umsatz mit 1,2 bis 1,8, bevorzugt 1,5 bis 1,7 Mol Brom pro Mol Ausgangsprodukt ein Bromierungsgemisch erhalten werden, das etwa 40 bis 85 Gew.-%, bevorzugt 80 bis 85 Gew.-% des jeweiligen Benzalbromids neben etwa 15 bis 60 Gew.-%, bevorzugt 15 bis 20 Gew.-%, des jeweiligen Benzylbromids enthält.

Soll beispielsweise das gegebenenfalls substiuierte Fluorbenzalbromid als Hauptprodukt erhalten werden, so kann man zur Erhöhung von dessen Ausbeute beispielsweise einen Teil des Bromierungsgemisches, der etwa dem Anteil am zugehörigen Benzylbromid entspricht, beispielsweise durch Destillation abtrennen und zur weiteren Bromierung wieder in das Ausgangsgemisch zurückführen.

Durch diese Verfahrensvariante gelingt es, die Ausbeute an dem jeweiligen Benzalbromid auf etwa 95 bis nahe 100 Gew.-% des Gesamtgewichts des Bromierungsgemisches zu steigern. Daneben kann dieses Gemisch noch fast 0 bis etwa 5 Gew.-%, bevorzugt weniger als 1 Gew.-%, besonders bevorzugt weniger als 0,1 Gew.-%, an dem jeweiligen Benzylbromid enthalten.

Weiterhin ist es möglich, 1 Mol gegebenenfalls substituiertes Fluortoluol mit etwa 2,2 bis 3,2 Mol, bevorzugt 2,3 bis 2,7 Mol, besonders bevorzugt 2,4 bis 2,5 Mol, Brom umzusetzen. Die dabei erhaltenen Reaktionsgemische können beispielsweise 15 bis 95 Gew.-%, bevorzugt 20 bis 80 Gew.-%, besonders bevorzugt 30 bis 60 Gew.-%,

an gegebenenfalls substituiertem Fluorbenzotribromid und beispielsweise etwa 5 bis 85 Gew.-%, bevorzugt 20 bis 80 Gew.-%, besonders bevorzugt 40 bis 70 Gew.-%, an gegebenenfalls substituiertem Fluorbenzalbromid enthalten.

Das Brom kann im erfindungsgemäßen Verfahren gasförmig, flüssig oder als Lösung zugegeben werden. Für den Fall, daß das Brom gasförmig zugegeben wird, kann dies mit oder ohne Verdünnung durch ein Inertgas durchgeführt werden. Beispiele für geeignete Inertgase sind Luft, Stickstoff, Wasserstoff, Kohlendioxid, Argon, Neon, Bromwasserstoff und Schwefeldioxid. Für den Fall, daß das Brom als Lösung zugegeben wird, kann beispielsweise eines der unten aufgeführten Lösungsmittel verwendet werden. Bevorzugt ist die Zugabe von flüssigem Brom ohne Lösungs- oder Verdünnungsmittel.

Das erfindungsgemäße Verfahren wird beispielsweise bei einer Temperatur von 130 bis 250°C, bevorzugt 150 bis 220°C, besonders bevorzugt 170 bis 210°C, durchgeführt.

Für den Fall, daß in Gegenwart eines Lösungsmittels gearbeitet wird, seien als inerte Lösungsmittel beispielsweise halogenierte aliphatische Kohlenwasserstoffe mit 1 bis 5 Kohlenstoffatomen, wie Methylenchlorid, Chloroform, Tetrachlorkohlenstoff, Dichlorethan, Trichlorethan, Tetrachlorethan und Hexachlorethan, bevorzugt Tetrachlorkohlenstoff, sowie aromatische Kohlenwasserstoffe und deren Halogenderivate, wie Benzol, Chlorbenzol, Brombenzol, Dichlorbenzol, Trichlorbenzol oder Tetrachlorbenzol, bevorzugt Chlorbenzol und Dichlorbenzol, genannt. Solche Lösungsmittel

können sowohl einzeln als auch im Gemisch miteinander für das erfindungsgemäße Verfahren eingesetzt werden. Sie können beispielsweise in einer Menge von bis zu 10 Vol.-Tl., bevorzugt bis zu 4 Vol.-Tl., besonders bevorzugt bis zu 1 Vol.-Tl., bezogen auf 1 Vol.-Tl. gegebenenfalls substituiertes Fluortoluol, eingesetzt werden.

Es ist jedoch auch möglich, das erfindungsgemäße Verfahren ohne Verwendung eines Lösungs- oder Verdünnungsmittels durchzuführen. Wegen der Vermeidung zusätzlicher Trennoperationen ist die Variante ohne zusätzliches Lösungsmittel bevorzugt.

Das erfindungsgemäße Verfahren kann unter photochemischer Initiierung, beispielsweise unter Einwirkung von natürlichem oder künstlichem Licht, oder unter radikalischer Initiierung, beispielsweise durch Einsatz von dem Fachmann bekannten Radikalbildnern, durchgeführt werden. Es ist jedoch auch möglich, das erfindungsgemäße Verfahren ohne Benutzung von photochemischer oder radikalischer Initiierung durchzuführen. Dieser zuletzt erwähnte Ausschluß einer photochemischen oder radikalischen Initiierung ist bevorzugt, da er apparative Vorteile durch den Fortfall einer Bestrahlungsmöglichkeit und durch den Fortfall einer zusätzlichen Dosiereinrichtung für einen etwa zu verwendenden Radikalbildner bringt und keine Rückstände eines etwa eingesetzten Radikalbildners im Produkt verursacht.

Bei der Reaktion des erfindungsgemäßen Verfahrens entsteht gleichzeitig Bromwasserstoff. Dieser wird kontinuierlich aus dem Reaktionssystem abgezogen. Dies geschieht im allgemeinen, wenn bevorzugterweise bei

Normaldruck gearbeitet wird, über den aufgesetzten Rückflußkühler und eine nachgeschaltete Absorptionseinrichtung. Für den Fall, daß unter erhöhtem Druck gearbeitet wird, etwa um ein tiefer siedendes Lösungsmittel aus der Gruppe der oben erwähnten Lösungsmittel einzusetzen, wird der Bromwasserstoff über eine Druckschleuse am Kopf des Rückflußkühlers aus dem Reaktor entnommen. Gegen Ende der Reaktion kann es vorteilhaft sein, zur vollständigen Entfernung des Bromwasserstoffs einen Unterdruck an den Reaktor anzulegen, beispielsweise im Bereich von 0,4 bis 0,95 bar.

Im allgemeinen wird das erfindungsgemäße Verfahren so durchgeführt, daß man das gegebenenfalls substituierte Fluortoluol, gegebenenfalls auch in einer technischen Qualität mit einem Gehalt von etwa 95 bis 99 Gew.-% an dem gewünschten gegebenenfalls substituierten Fluortoluol, gegebenenfalls mit einem inerten Lösungsmittel auf etwa 160 bis 190°C aufheizt und bei guter Durchmischung die vorgesehene Menge an Brom in der Weise eindosiert, daß es unterhalb der Oberfläche des Reaktionsgemisches, zweckmäßigerweise in der unteren Hälfte des Reaktionsgemisches, beispielsweise in der Nähe des Bodens des Reaktionsgefäßes, eintritt. Dies hat den Vorteil, daß praktisch kein Brom aus dem Reaktionsgemisch entweicht. Es ist auch möglich, nur einen Teil des gegebenenfalls substituierten Fluortoluols vorzulegen und das restliche Fluortoluol simultan mit dem Brom in den Reaktor einzudosieren, wobei die simultane Zugabe sowohl chargenweise als auch kontinuierlich durchgeführt werden kann. Nach Zugabe der vorgesehenen Menge an Brom wird bis zum Ende der Bromwasserstoffentwicklung nachgerührt.

Für den Fall, daß das bei der α-Bromierung erhaltene Gemisch weiter verarbeitet werden soll, ist eine vorherige Aufarbeitung des Reaktionsgemisches bei lösungsmittelfreier Arbeitsweise vor der Weiterverarbeitung im allgemeinen nicht erforderlich. Beispielsweise kann ein Reaktionsgemisch, das 90 bis 93 Gew.-% gegebenenfalls substituiertes Fluorbenzalbromid enthält, in dieser Form, gegebenenfalls nach Entfernung von restlichem Bromwasserstoff im Vakuum, direkt weiter verarbeitet werden. Bei Bedarf kann selbstverständlich eine weitere Reinigung oder auch eine Fraktionierung des Reaktionsgemisches durch Destillation, Vakuumdestillation oder Umkristallisation erfolgen.

Das erfindungsgemäße Verfahren kann sowohl kontinuierlich als auch diskontinuierlich durchgeführt werden.

Die Erfindung betrifft weiterhin Gemische, die 90 bis 93 Gew.-% gegebenenfalls substituiertes Fluorbenzalbromid, 3 bis 4 Gew.-% gegebenenfalls substituiertes Fluorbenzylbromid und 4 bis 6 Gew.-% gegebenenfalls substituiertes Fluorbenzotribromid enthalten, wobei die Gewichtsprozente sich auf das Gesamtgewicht der Gemische beziehen.

Die Erfindung betrifft weiterhin Gemische, die 15 bis 95 Gew.-% gegebenenfalls substituiertes Fluorbenzotribromid und 5 bis 85 Gew.-% gegebenenfalls substituiertes Fluorbenzalbromid enthalten, wobei sich die Gewichtsprozente auf das Gesamtgewicht der Gemische beziehen.

Die Erfindung betrifft weiterhin Gemische, die 40 bis 85 Gew.-% gegebenenfalls substituiertes Fluorbenzalbromid und 15 bis 60 Gew.-% gegebenenfalls substituiertes Fluorbenzylbromid enthalten, wobei die Gewichtsprozente sich auf das Gesamtgewicht der Gemische beziehen.

Die Erfindung betrifft weiterhin Gemische, die 95 bis nahe 100 Gew.-% gegebenenfalls substituiertes Fluorbenzalbromid und nahezu 0 bis etwa 5 Gew.-% gegebenenfalls substituiertes Fluorbenzylbromid enthalten, wobei die Gewichtsprozente auf das Gesamtgewicht der Gemische bezogen sind.

Die genannten Gemische sind wertvolle Zwischenprodukte, die durch Verseifung des in die Methylgruppe erfindungsgemäß eingetretenen Broms den Zugang zu wertvollen Verbindungen eröffnen. So kann beispielsweise ein erfindungsgemäß erhältliches substituiertes Benzalbromid oder die erfindungsgemäßen Gemische, die überwiegend Benzalbromid enthalten, durch Verseifung in die zugehörigen substituierten Benzaldehyde umgewandelt werden. Diese Benzaldehyde sind beispielsweise wertvolle Ausgangsprodukte für Riechstoffe. Weiterhin können die erfindungsgemäß erhältlichen substituierten Benzylbromide in die zugehörigen Benzylalkohole umgewandelt werden, die durch Veresterung ebenfalls zu Riech- oder Aromastoffen, zu Pharmazeutika und anderen Wirkstoffen, wie Insektiziden, Fungiziden und Bakteriziden umgewandelt werden können. Die erfindungsgemäß erhältlichen gegebenenfalls substituierten Fluorbenzotribromide bzw. die erfindungsgemäßen Gemische mit einem überwiegenden Gehalt an Benzotribromid können beispielsweise durch

Verseifung in die zugehörigen substituierten Benzoesäuren oder Benzoesäurebromide umgewandelt werden, die ebenfalls wertvolle Ausgangsstoffe für die Herstellung von Riechstoffen, biologischen Wirkstoffen, wie Pharmazeutika, Insektiziden, Bakteriziden und Fungiziden, oder für den Fall der Benzoesäuren als Komponente für Lackrohstoffe und Alkydharze verwendbar sind.

Die Verseifung der erfindungsgemäßen Gemische bzw. erfindungsgemäß erhältlichen α-bromierten Verbindungen kann beispielsweise mit Säuren, wie 50 bis 90 gew.-%iger Schwefelsäure oder 50 bis 90 gew.-%iger Ameisensäure bei erhöhter Temperatur oder mit Wasser in Gegenwart von alkalisch reagierenden Stoffen durchgeführt werden.

Das erfindungsgemäße Verfahren liefert die α-bromierten Verbindungen in hoher Selektivität und in guten Ausbeuten. Das Verfahren ist technisch einfach zu handhaben, was insbesondere auf die bevorzugten Varianten ohne photochemische oder radikalische Initiierung und weiterhin auf die bevorzugte Variante ohne Verwendung eines Lösungsmittels zutrifft.

Beispiel 1

In einer Apparatur, die aus einem Rührkolben mit Thermometer, Rückflußkühler und einem bis zum Boden reichenden Tropftrichter besteht, wurden 283,5 g (1,5 Mol) 3-Brom-4-fluortoluol bei 180°C vorgelegt und dann unter Rühren 499 g (3,12 Mol) Brom in einer solchen Geschwindigkeit unter die Oberfläche getropft, daß im Rückflußkühler keine braunen Bromdämpfe sichtbar waren. Die Temperatur wurde nach Beginn des Zutropfens auf 200°C gesteigert und während der Zutropfzeit von 2 1/4 Stunde dabei gehalten. Die Bromwasserstoffentwicklung setzt sofort ein. Der Kolbeninhalt färbt sich im Verlaufe der Reaktion dunkelbraun. Nach Beendigung der Bromwasserstoffentwicklung und Abkühlung auf Raumtemperatur werden 500 ml $H_2O$ zugegeben, die mit wenig Sodalösung neutral gestellt werden. Nach Abtrennung der Wasserphase wird die organische Phase nochmals mit der gleichen Menge Wasser gewaschen und nach deren Abtrennung über Kaliumcarbonat getrocknet. Es werden 521,9 g Bromierungsgemisch erhalten, das nach gaschromatographischer Analyse zu 90,7 Gew.-% aus 3-Brom-4-fluorbenzalbromid, zu 2,5 Gew.-% aus dem zugehörigen Benzylbromid und zu 6,6 Gew.-% aus dem zugehörigen Benzotribromid besteht.

Beispiel 2 (zur Verwendung)

506,5 g (1,32 Mol) 3-Brom-4-fluorbenzalbromid werden mit 237 g (4,38 Mol) 85 gew.-%iger Ameisensäure bei Rückflußbedingungen (etwa 105 bis 120°C) während 14 Stunden erhitzt. Anschließend wird die organische Phase abgetrennt, mit 500 ml Wasser durchgerührt und

mit konzentrierter wäßriger Sodalösung neutral gestellt. Die organische Phase wird abgetrennt und über Natriumsulfat getrocknet. Zur Erhöhung der Ausbeute wird die wäßrige Phase mit wenig Toluol ausgeschüttelt und das Trockenmittel Natriumsulfat ebenfalls mit Toluol gewaschen. Die erhaltenen Lösungen in Toluol werden vereinigt und vom Toluol befreit. Der Gesamtrückstand, der erhalten wurde, beträgt 272,3 g und enthält nach gaschromatographischer Analyse 91,2 Gew.-% 3-Brom-4-fluorbenzaldehyd, 4,2 Gew.-% 3-Brom-4-fluorbenzoesäure und 3,9 Gew.-% 3-Brom-4-fluor-benzylbromid. Die Ausbeute beträgt 92,7 % der theoretischen Ausbeute an 3-Brom-4-fluorbenzaldehyd, bezogen auf das eingesetzte Benzalbromid. Beim Ansäuern der mit Soda neutralisierten wäßrigen Phase werden noch 24 g 3-Brom-4-fluorbenzoesäure vom Schmelzpunkt 137 bis 139°C isoliert.

Beispiel 3

In der Apparatur aus Beispiel 1 werden 110,1 g (1,0 Mol) 4-Fluortoluol zum Sieden (Kp. 116°C) erhitzt und unter Rühren in 7 Stunden 319,6 g (2,0 Mol) Brom zugegeben. Die Wärmezufuhr wird so reguliert, daß bis zum Erreichen von 200°C stets die maximale Sumpftemperatur herrscht. Die Temperatur von 200°C wird gehalten und bei dieser Temperatur noch eine halbe Stunde nachgerührt. Man erhält 263,3 g dunkelbraunes Öl, das nach gaschromatographischer Analyse zu 90,9 Gew.-% aus 4-Fluorbenzalbromid, zu 3,7 Gew.-% aus 4-Fluorbenzylbromid und zu 4,8 Gew.-% aus 4-Fluorbenzotribromid besteht.

Beispiel 4 (zur Verwendung)

Zu dem im Beispiel 3 erhaltenen Gemisch wird in der gleichen Apparatur 5 g eines stark sauren Sulfonsäuregruppen enthaltenden Styrol-Divinylbenzol-Ionenaustauschers (Lewatit S 100 der Fa. Bayer AG) hinzugefügt und bei 80°C unter starkem Rühren 108,2 g (2,0 Mol) 85 %ige Ameisensäure zugetropft. Im Verlauf von 4 Stunden steigert man die Sumpftemperatur auf 120°C bis die Gasentwicklung beendet ist. Nach dem Abkühlen saugt man vom Ionenaustauscher und ausgefallener 4-Fluorbenzoesäure ab und destilliert den Rückstand über eine Füllkörperkolonne. Es wird ein Hauptlauf von 100,9 g bei 70 bis 80°C und 26 mb erhalten, der nach gaschromatographischer Analyse zu 98,1 Gew-% aus 4-Fluorbenzaldehyd besteht.

Aus dem Filterkuchen werden der wiederverwendbare Ionenaustauscher und 6,7 g 4-Fluorbenzoesäure mit Fp. 181 bis 183°C erhalten.

Patentansprüche

1) Verfahren zur α-Bromierung von gegebenenfalls substituierten Fluortoluolen, dadurch gekennzeichnet, daß man Fluortoluole der Formel

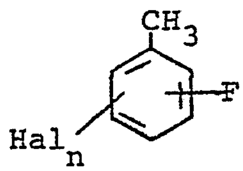

,

in der

Hal für Fluor, Chlor oder Brom steht und
n gleich 0 oder 1 ist,

mit 0,5 bis 3,2 Mol Brom pro Mol Fluortoluol bei einer Temperatur von 130 bis 250°C, gegebenenfalls in Gegenwart eines Lösungsmittels und gegebenenfalls unter photochemischer oder radikalischer Initiierung umsetzt und den gleichzeitig gebildeten Bromwasserstoff kontinuierlich aus dem Reaktionsgemisch entfernt.

2) Verfahren nach Anspruch 1, dadurch gekennzeichnet, daß man in Gegenwart von bis zu 10 Vol.-Tl. eines inerten Lösungsmittels pro 1 Vol.-Tl. Fluortoluol arbeitet.

3) Verfahren nach Anspruch 1 und 2, dadurch gekennzeichnet, daß unter Ausschluß von photochemischer oder radikalischer Initiierung gearbeitet wird.

4) Verfahren nach Anspruch 1 bis 3, dadurch gekennzeichnet, daß mit 1,9 bis 2,2 Mol Brom gearbeitet wird.

5) Verfahren nach Anspruch 1 bis 3, dadurch gekennzeichnet, daß mit 2,2 bis 3,2 Mol Brom gearbeitet wird.

6) Verfahren nach Anspruch 1 bis 3, dadurch gekennzeichnet, daß mit 0,5 bis 1,9 Mol Brom gearbeitet wird.

7) Gemische, enthaltend 90 bis 93 Gew.-% gegebenenfalls substituiertes Fluorbenzalbromid, 3 bis 4 Gew.-% gegebenenfalls substituiertes Fluorbenzylbromid und 4 bis 6 Gew.-% gegebenenfalls substituiertes Fluorbenzotribromid, alles bezogen auf das Gesamtgewicht der Gemische.

8) Gemische, enthaltend 15 bis 95 Gew.-% gegebenenfalls substituiertes Fluorbenzotribromid und 5 bis 85 Gew.-% gegebenenfalls substituiertes Fluorbenzalbromid.

9) Gemische, enthaltend 40 bis 85 Gew.-% gegebenenfalls substituiertes Fluorbenzalbromid und 15 bis 60 Gew.-% gegebenenfalls substituiertes Fluorbenzylbromid.

10) Gemische, enthaltend 95 bis 100 Gew.-% gegebenenfalls substituiertes Fluorbenzalbromid und 0 bis 5 Gew.-% gegebenenfalls substituiertes Fluorbenzylbromid.

0045430

Europäisches Patentamt

# EUROPÄISCHER RECHERCHENBERICHT

Nummer der Anmeldung
EP 81 10 5681

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A - 1 815 452 (MERCK & CO.)<br>* Seiten 34-36; Beispiel 1; Tabelle 1 *<br>-- | 1,2 | C 07 C 25/13<br>17/14 |
| A | DE - A - 2 600 182 (SPOFA VEREINIGTE PHARMAZEUTISCHE WERKE)<br>* Seite 58, letzter Absatz *<br>-- | 1 | |
| A | DE - A - 2 855 703 (SPOFA SPOJENE PODNIKY PRO ZDRAVOTNICKOU VYROOBU)<br>* Seite 51, letzter Absatz *<br>-- | 1 | RECHERCHIERTE SACHGEBIETE (Int. Cl.³)<br>C 07 C 25/13<br>17/14 |
| A | DE - A - 2 941 312 (SHELL)<br>* Beispiel 1a *<br>----- | 1 | |

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

✓ Der vorliegende Recherchenbericht wurde für alle Patentansprüche erstellt.

| Recherchenort | Abschlußdatum der Recherche | Prüfer |
|---|---|---|
| Den Haag | 14-10-1981 | VAN GEYT |